# EUROPEAN PATENT SPECIFICATION

(11) **EP 1 226 234 B1**
(45) Date of publication and mention of the grant of the patent: **29.06.2011**
(21) Application number: 00972990.6
(22) Date of filing: 01.11.2000
(51) Int. Cl.: C12N 5/071, A61K 35/36, A61P 17/14

(54) **HAIR TRANSPLANTATION**
HAARTRANSPLANTATION
TRANSPLANTATION DE CHEVEUX

(30) Priority: 03.11.1999 GB 9925964
(43) Date of publication of application: 31.07.2002
(73) Proprietor: Reynolds, Amanda, Jane, Richmond North Yorkshire DL10 7AU (GB); Jahoda, Colin Albert Buchanan, Richmond, North Yorkshire DL10 7AU (GB)
(72) Inventor: Reynolds, Amanda, Jane, Richmond North Yorkshire DL10 7AU (GB); Jahoda, Colin Albert Buchanan, Richmond, North Yorkshire DL10 7AU (GB)
(74) Representative: Taylor, Kate Laura
(86) International application number: PCT/GB2000/004177
(87) International publication number: WO 2001/032840

(56) References cited:
- JAHODA C A ET AL: "Human hair follicle regeneration following amputation and grafting into the nude mouse." JOURNAL OF INVESTIGATIVE DERMATOLOGY, (1996 DEC) 107 (6) 804-7. , XP001010408
- JAHODA C A ET AL: "Dermal-epidermal interactions --follicle-derived cell populations in the study of hair-growth mechanisms." JOURNAL OF INVESTIGATIVE DERMATOLOGY, (1993 JUL) 101 (1 SUPPL) 33S-38S. REF: 39 , XP001010322
- REYNOLDS A J ET AL: "Cultured dermal papilla cells induce follicle formation and hair growth by transdifferentiation of an adult epidermis." DEVELOPMENT, (1992 JUN) 115 (2) 587-93. , XP001010312
- DATABASE BIOSIS [Online] BIOSCIENCES INFORMATION SERVICE, PHILADELPHIA, PA, US; PISANSARAKIT P ET AL: "INDUCTION OF HAIR FOLLICLES IN MOUSE SKIN BY RAT VIBRISSA DERMAL PAPILLAE." retrieved from STN Database accession no. 1986340148 XP002179721 & J EMBRYOL EXP MORPHOL, (1986) 94 (0), 113-120. ,
- DATABASE BIOSIS [Online] BIOSCIENCES INFORMATION SERVICE, PHILADELPHIA, PA, US; WORST P K M ET AL: "REFORMATION OF ORGANIZED EPIDERMAL STRUCTURE BY TRANSPLANTATION OF SUSPENSIONS AND CULTURES OF EPIDERMAL AND DERMAL CELLS." retrieved from STN Database accession no. 1983199667 XP002179722 & CELL TISSUE RES, (1982) 225 (1), 65-78. ,
- DATABASE BIOSIS [Online] BIOSCIENCES INFORMATION SERVICE, PHILADELPHIA, PA, US; WU, JINJIN ET AL: "An efficient method for isolation and cultivation of human scalp dermal papilla cells." retrieved from STN Database accession no. 1998181268 XP002179723 & ZHONGHUA PIFUKE ZAZHI, (DEC., 1997) VOL. 30, NO. 6, PP. 383-385. ,

## Description

The invention relates to the use of dermal sheath tissue in hair transplantation therapy and a method to isolate dermal sheath cells showing immune privilege.

Adult hair growth and cyclic hair follicle regeneration are driven by exquisitely regulated dermal-epidermal tissue interactions at the follicle base (1,2). In rodents, isolated hair follicle dermal cells can induce new follicles when re-introduced *in vivo* (3,4), however, equivalent properties have never been demonstrated in humans. Despite great advances in the field of human tissue and organ transplantation, the ability to transfer cells from one individual to another irrespective of immunocompatibility has not yet been achieved.

Skin is a highly complex organ covering the external surface of the body and merging, at various body openings, with the mucous membranes of the alimentary and other canals. It has multiple functions such as preventing water loss from the body, but predominantly acts as a protective barrier against the action of physical, chemical and bacterial agents on deeper tissues. Skin is elastic and except for a few areas such as the palms, soles and ears it is loosely attached to underlying tissue. It varies in thickness from 0.5mm (0.02 inches) on the eyelids to 4mm (0.17 inches) or more on the palms and soles.

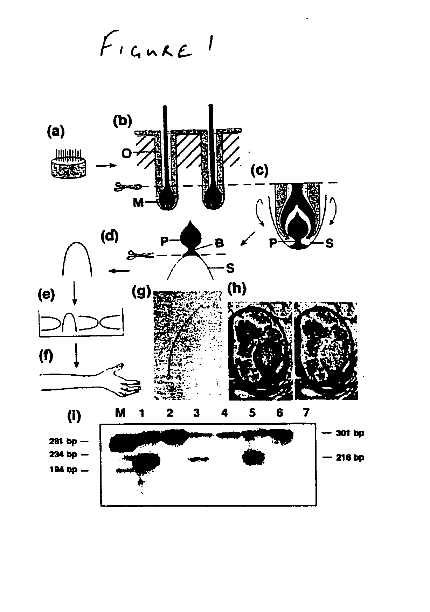

Skin is composed of two layers (please refer to Figure 1 which illustrates an anatomical cross-sectional view through a slice of skin), the outer layer, which is comparatively thin (0.1 mm) is called the epidermis, or cuticle, it is several cells thick and has an external, horny layer of dead cells that are constantly shed from the surface and replaced from below by a basal layer of cells, called the stratum germinativum. The epidermis is composed predominantly of keratinocytes which make up over 95% of the cell population, the rest include dendritic cells such as Langerhans cells and melanocytes. It is essentially cellular and non-vascular, there being relatively little extracellular matrix except for the layer of collagen and other proteins beneath the basal layer of keratinocytes. Keratinocytes of the basal layer are constantly dividing, and daughter cells subsequently move outwards, where they undergo a period of differentiation and are eventually sloughed off from the surface. The inner layer of the skin is called the dermis and is composed of a network of collagenous extracellular material, elastic fibres, blood vessels and nerves. Contained within it are hair follicles with associated sebaceous glands. This is collectively known as the pilosebaceous unit (PU).

Hair growth can be looked on as stimulation, augmentation or regeneration by or of the PU. PU stimulation can result in increased hair fibre output from PU by elevating productivity and/or altering hair cycle behaviour, for instance, promoting the active (anagen) over the inactive (telogen) phase. PU augmentation can result in increased hair output by addition to (e.g. cell incorporation), or support of (e.g. preventing deterioration or cell loss from), an existing PU to effect hair production increase and/or maintenance, with or without PU size and/or cycle changes. PU regeneration can result in increased hair output by restoration of an existing PU, or part thereof, to a productive status, with or without PU size and/or cycle changes.

The interface between the epidermis and dermis is extremely irregular and consists of a succession of papillae, or finger like projections. Beneath the basal epidermal cells along this interface the specialised extracellular matrix is organised into a distinct structure called the basement membrane.

The mammalian hair fibre is the product of a small peg of tissue known as the hair follicle which lies immediately underneath the skin's surface. The distal part of said follicle is in direct continuation with the cutaneous epidermis externally. Although small, the follicle comprises a highly organised system of recognisably different layers arranged in concentric series. Active hair follicles extend down through the dermis, the hypodermis (a loose layer of connective tissue), and the fat or adipose layer.

At the base of any active follicle lies the hair bulb, which consists of a body of dermal cells, known as the dermal papilla, contained in an inverted cup of epidermal cells known as the epidermal matrix (please refer to Figure 1). Irrespective of follicle type, the hair fibre, together with several supportive epidermal layers, is produced by germinative epidermal cells at the very base of this epidermal matrix. The lowermost dermal sheath is contiguous with the papilla basal stalk, from where it curves externally around all of the epidermal layers of hair matrix as a thin covering of tissue, and then continues as a tube or sleeve for the length of the follicle.

We here describe a population of cells which show immunoprivilege and that these cells have utility with regard to providing cellular compositions for use in hair transplant therapy.

According to a first aspect of the invention there is provided a method for the preparation of a medicament for the transplantation of dermal sheath cells/tissue in hair replacement therapy comprising:
i) providing at least one dermal sheath cell, the donating cell;
ii) the donating cell being later inserted at at least one selected site in an animal, the recipient animal.

Hair replacement therapy is intended to include all modes of replacing or restoring hair that was once present, as well as, creating or stimulating increased hair mass in any body site where it is either absent or considered to be sparce using dermal sheath cells/tissue. Hair replacement therefore relates to any mode of effecting increased hair mass via pilosebaceous unit stimulation and/or augmentation and/or regeneration and/or induction, with or without pilosebaceous unit size and/or hair cycle behaviour change using dermal sheath cells/tissue.

Preferably the donating cell and the recipient animal are the same gender.

Alternatively the donating cell is transplanted into an animal of opposite gender. Preferably the donating cell is derived from a male and the recipient animal is female.

More ideally still said donating cell is derived from a female and the recipient animal is male.

The presently available drugs are only suitable for use by a restricted proportion of those who suffer mild to moderate alopecia, and only a subgroup of those treated will respond. No current drug can treat significant hair loss and none is able to reverse the process at all, at best, there may be some temporary " slowing down".

In a further preferred method of the invention said animal is human.

According to a further aspect of the invention there is provided a composition comprising at least one dermal sheath cell/tissue for use in hair transplantation.

In a preferred embodiment of the invention the cell is derived from one gender type. Preferably said gender is male. Alternatively the gender is female. More ideally still said composition is for use in transplantation into an animal of opposite gender to that from which the donating cell(s) have been derived. Preferably said animal is human.

In yet a further preferred embodiment of the invention said composition is for use in the treatment of diseases resulting in hair loss, for example and not by way of limitation, alopecia.

Alopecia is a generic term which encompasses all forms of medical or cosmetic hair loss. Alopecia may be due to follicle absence, destruction (which may be scarring), disease, infection, shrinkage, disregulation (hair cycle alterations), or complete shut down/dormancy. These changes can be temporary or permanent, partial, regional and/or apparently total.

Examples of conditions which would be considered under this term are: hypopituitarism, e.g. Simmonds' disease and Sheehan's syndrome; other endocrinopathies e.g. hypothyroidism, diabetes mellitus, adrenal dyperplasia; chronic disease, e.g. lupus erythematosus, neoplasma; monilethrix; pseudomilethrix; heredilary hypotrichosis simplex of the scalp; congenital triangular temporal alopecia; alopecia of the cranial sutures - Hallermann Streiff's syndrome; hereditary cicatrical alopecia, e.g. Marie Unna type generalised hypotrichosis; diffuse congenital scarring follicular keratosis; epidermal nevus - benign epithelial hamartoma and/or malignant tumour or benign ;physical trauma, e.g. car accident, sporting injury; post-surgical; anti-tumoural agents, e.g. cytostatic and/or alkylating agents;chemical trauma, e.g. thioglycolates, anti-metabolics; ionizing radiation; toxic drugs, e.g. thallium, vitamin A; self-inflicted physical trauma, e.g. Trichotillomania; bacterial, e.g. Pyodermitis, Bockhart's impetigo; mycotic/fungal, e.g. candidiasis, dermatophylosia; accidental trauma/rubbing, e.g. hats, hairdressers etc. A related term is hypotrichosis which is defined as a decrease in the production of hair.

In further preferred embodiment said composition is for use in conditions where a therapeutic treatment results in permanent hair loss, for example and not by way of limitation, cancer treatment.

In yet still a further preferred embodiment of the invention said composition is for use in the cosmetic treatment of hair loss.

Cosmetic treatment of hair loss encompasses male-pattern baldness, and female male-pattern baldness, of the scalp. This relates to hair loss as a consequence of age-onset processes/deterioration, without any obvious pathology.

According to yet still a further preferred embodiment of the invention there is provided a method for the isolation of dermal sheath cells comprising:
i) isolation of dermal sheath cells/tissue from a sample of skin tissue; and, optionally, storing said dermal sheath cells/tissue prior to use.

In the invention said sample is substantially devoid of cells involved in a recipient's rejection response. Preferably said sample is devoid of leukocytes and/or Langerhans cells. More preferably still said sample is devoid of blood vessel endothelial cells.

In a preferred method of the invention said skin tissue is isolated from a particular gender type.

In yet a further preferred method of the invention said storage is by cryopreservation.

According to a further aspect of the invention there is provided a method to isolate dermal sheath tissue/cells showing immune privilege comprising:
i) rapid amputation of hair follicle end bulbs from a punch biopsie sample obtained from an animal skin;
ii) eversion and exposure of epidermal components which are scraped away and discarded;
iii) separation of dermal sheath/cells and dermal papillae;
iv) dermal sheath tissue/cells are cleaned; and optionally
v) dermal sheath tissue/cells are pooled separately in culture medium.

An embodiment of the invention will now be described, by example only and with reference to the following figures;
Figure 1 a-f is a representation of the transplantation regime for implanting male dermal sheath tissue into a female recipient;

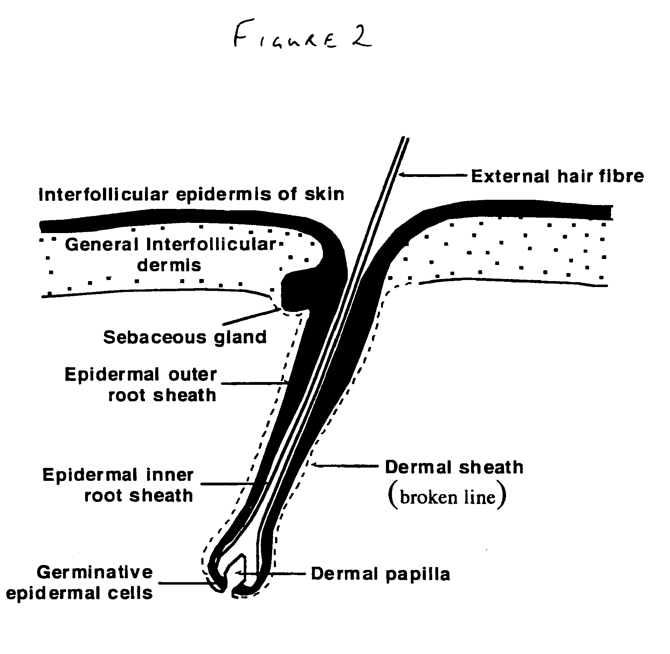

Figure 2 is a graphical representation of a hair follicle.

### Materials and Methods

Methods and analysis of follicle induction. Punch biopsies were taken from male scalp skin (a) and their hair follicle end bulbs rapidly amputated (b), everted (c) and the exposed epidermal components scraped away and discarded (d). The dermal sheath and dermal papillae were separated, cleaned, and pooled separately in culture medium (e). Up to 11 pieces of dermal sheath were combined and implanted into small, shallow wounds on the inner forearm (f). Induced fibres were distinct and often pigmented (g). Laser capture microdissection (Pixi Cell, Arcturus Engineering Inc.California) was used to isolate cells from de-coverslipped histology slides. From induced follicles (h) an estimated 200 to 400 dermal papilla cells were first targeted and then removed (arrows) with 6 "punches". Equivalent (or greater) numbers of cells were dissected from controls. Following DNA extraction, for determination of the X and Y chromosome complement, we performed PCR based gender determination by restriction analysis of simultaneously amplified ZFX and ZFY sequences, as previously described in detail (7). Due to the limited amount of template DNA, digested PCR products were Southern blotted (see below) and hybridized with a probe consisting of the same PCR product for visualisation. Southern analysis of the restricted PCR products (i) shows two products with molecular weights of 301 and 216 base pairs, indicative of the X and Y chromosomes respectively, in DNA extracted from blood from the male donor [lane1], from the laser capture microdissected cells of the induced follicle above [lane 3], and from laser captured skin cells of an unrelated male [lane 5]. A single band of 301 bp reflecting only the presence of the X chromosome, is visible in DNA extracted from the blood of the female host [lane 2], from laser capture microdissected cells of the female host adjacent to the induced follicle in (a)[lane 4], and from laser captured skin cells from an unrelated female [lane 6]. Lane 7 is a no DNA control. Lane M contains the molecular weight markers x 174 digested with Hae III. The representative 281/271 doublet, 234 and 194 base pair bands are shown. Key: epidermal matrix (M), inner root sheath (I), outer root sheath (O), dermal papilla (P), basal stalk (B), dermal sheath (S).

### Results

To test the inductive and immunoreactive properties of human hair follicle dermis, we microdissected dermal sheath tissue from the bases of scalp skin follicles of one of us, a male. We implanted this into shallow skin wounds on the inner forearm of another of us, a genetically unrelated and immunologically incompatible female recipient (Fig. 1a-f). Five months later, the same female host received follicle dermal sheath and dermal papilla grafts from the same male donor, and subsequently, in a third experiment, dermal sheath from a second unrelated donor male. All of the wound sites healed rapidly and, importantly, lacked any overt inflammatory reaction. Remarkably, each of the dermal sheath implantation sites produced new follicles and fibres 3 to 5 weeks post-operatively (Fig.1g). Unlike the tiny unpigmented local vellus hairs, those which had been newly induced were larger, thicker, mostly pigmented, and grew in variable directions. None of the new follicles showed evidence of rejection when biopsied between 41 and 77 days post-operatively. Histology confirmed that the new follicles were morphologically normal, with oval dermal papillae overlaid by a pigmented epidermal matrix at their base. We only observed a light inflammatory infiltrate around the upper regions of one specimen.

To directly demonstrate that the follicles were newly induced by the implanted human dermal tissue we analysed DNA from our histological specimens. We employed laser capture microdissection (LCM) (6) to isolate dermal papilla cells from one of the follicles (Fig.1h). We then performed PCR-based gender determination and subsequent restriction analysis of simultaneously amplified ZFX and ZFY sequences (7). This confirmed that the DNA extracted from the papilla cells had an X and Y complement (male), whereas cells taken from other regions of the same follicle and skin epidermis in the immediate vicinity had only X chromosome complement (female) (Fig.1,i).

### Discussion

We have shown that adult human male scalp follicle dermal sheath tissue, in numerical terms perhaps only a few hundred cells, formed a new dermal papilla and induced new hair follicles in genetically unrelated host female skin. Moreover, these follicles survived prolonged periods without rejection. While the inductive capacity of dermal sheath cells represents a novel and intriguing finding, the failure of the dermal papillae to induce follicles is somewhat surprising, since in previous animal studies, the papilla has shown superior inductive powers (4).

Appendage formation requires a complex level of interaction between the host epidermal and donor dermal cells. For this to occur, the male tissue first must be tolerated. Donor and host were blood group and HLA disparate, yet we observed no strong evidence of graft rejection, even after repeat grafting when a rapid second-set rejection might have been expected.

Grafts are more likely to be tolerated if there is no coincidental transfer of passenger leukocytes which express the donor's class II MHC molecules, Langerhans cells or blood vessel endothelial cells, since all of these factors can stimulate the host's alloreactive response (8,9). In our protocol, the tissue was washed repeatedly to remove adherent cells, and was so discrete that it was unlikely to have included blood vessels. Microenvironmental tissue damage, which is also believed to contribute to the host's immune response was also negligible in our minimally invasive procedure. While these factors may have contributed to graft tolerance, the main reason probably lies with the tissue itself. Immunohistochemical examination of intact appendages in vivo suggests that the lowermost tissues of rat and human hair follicles exhibit special immune status (10). Our work provides further support for this, but more specifically, it suggests that the follicle end bulb dermal sheath is among a restricted group of immunoprivileged tissues that can themselves be transplanted to foreign sites without being rejected (11).

Our results show, for the first time, that adult human follicle dermal cells retain the remarkable capacity to initiate epithelial-mesenchymal interactions and create new follicles. The fact that the allografted dermal sheath cells were not rejected provides direct experimental evidence that they have privileged immune status.

### References

1. Hardy, M.H. Trends in Genetics. 8(2), 55-61(1992).
2. Oliver, R.F. J Soc Cosmet Chem 22, 741-755 (1971).
3. Reynolds, A.J., Jahoda, C.A.B. Development 115, 587-593 (1992).
4. Jahoda, C.A.B., Reynolds, A.J., Oliver, R.F. J Invest Dermatol .101, 584-590 (1993).
5. Tosti, A., Camacho-Martinez, F. Dawber, R. J Eur Acad Dermatol Venereol. 12, 205-214 (1999)
6. Chong, S.S., Kristjansson, K., Cota J., Handyside A.H., Hughes M.R. Hum. Molec. Genet. 2, 1187-1191 (1993).
7. Fabre, J.W., Milton A.D., Spencer, S., Settaf, A., Houssin D. Transplant Proc .19 (1), 45-49 (1987).
8. Halloran, P.F., Broski A.P., Batiuk T.D., Madrenas J.Transplant Immunology, 1(1), 3-27 (1993)
9. Ibrahim, M.A.A., Chain, B.M., Katz, D.R. Immunology Today 16, 181-186(1995)
10. Gibson, W.T., Westgate, G.E., Craggs, R.I. Annals NY Acad Sci USA 642, 291-300 (1991).
11. Simone, N.L., Bonner R.F., Gillespie, J.W., Emmert-Buck M.R. & Liotta L.A. Trends in Genetics .14,(7) 272-276 (1998).

## Claims

1. The use of isolated dermal sheath cells for the manufacture of a medicament for use in the treatment of conditions in a recipient animal which would benefit from hair replacement and/or augmentation.

2. The use according to Claim 1, wherein the isolated dermal sheath cells and recipient animal are the same gender.

3. The use according to Claim 1, wherein the isolated dermal sheath cells and recipient animal are different genders.

4. The use according to Claim 3, wherein the isolated dermal sheath cells are male and the recipient animal is female.

5. The use according to Claim 3, wherein the isolated dermal sheath cells are female and the recipient animal is male.

6. The use according to any of Claims 1 to 5, wherein the recipient animal is human.

7. The use of isolated dermal sheath cells for the manufacture of a medicament for the treatment of alopecia.

8. The use of isolated dermal sheath cells for the manufacture of a medicament for the treatment of hair loss as a result of cancer therapy.

9. The use of isolated dermal sheath cells for the cosmetic treatment of hair loss.

10. A method to isolate dermal sheath cells showing immune privilege comprising isolating dermal sheath cells from a sample of skin tissue removed from an animal such that the cells are devoid of immune cells involved in a recipient animals rejection response.

11. A method according to Claim 10, wherein the immune cells are leukocytes.

12. A method according to Claim 10, wherein the immune cells are Langerhans cells.

13. A method according to Claim 10, wherein the immune cells are blood vessel endothelial cells.

14. A method according to any of Claims 10-13, wherein the isolated dermal sheath cells are stored by cryopreservation.

15. A method according to any of Claims 10-14, wherein the isolated dermal sheath cells are derived from a female animal.

16. A method according to any of Claims 10-14, wherein the isolated dermal sheath cells are derived from a male animal.

17. A method according to any of Claims 10-16, wherein the animal is human.

18. A method according to claim 10 wherein said sample of skin is a punch biopsy and said dermal sheath cells are isolated by a method comprising:
i) rapid amputation of hair follicle end bulbs from said punch biopsy;
ii) eversion and exposure of epidermal components which are scraped away and discarded;
iii) separation of dermal sheath/cells and dermal papillae.
iv) dermal sheath tissue/cells are cleaned; and optionally;
v) dermal sheath tissue/cells are pooled separately in culture medium.

## Patentansprüche

1. Verwendung isolierter dermaler Hüllzellen zur Herstellung eines Medikaments zur Verwendung in der Behandlung von Krankheiten in einem Empfängertier, das von Haarersatz und/oder Haarvermehrung profitieren würde.

2. Die Verwendung gemäß Anspruch 1, wobei die isolierten dermalen Hüllzellen und das Empfängertier das gleiche Geschlecht haben.

3. Die Verwendung gemäß Anspruch 1, wobei die isolierten dermalen Hüllzellen und das Empfängertier verschiedene Geschlechter haben.

4. Die Verwendung gemäß Anspruch 3, wobei die isolierten dermalen Hüllzellen männlich sind und das Empfängertier weiblich ist.

5. Die Verwendung gemäß Anspruch 3, wobei die isolierten dermalen Hüllzellen weiblich sind und das Empfängertier männlich ist.

6. Die Verwendung gemäß einem der Ansprüche 1 bis 5, wobei das Empfängertier menschlich ist.

7. Verwendung isolierter dermaler Hüllzellen zur Herstellung eines Medikaments zur Behandlung von Alopezie.

8. Verwendung isolierter dermaler Hüllzellen zur Herstellung eines Medikaments zur Behandlung von Haarverlust als einer Folge von Krebstherapie.

9. Verwendung isolierter dermaler Hüllzellen zur kosmetischen Behandlung von Haarverlust.

10. Verfahren zum Isolieren immunprivilegierter dermaler Hüllzellen, umfassend das Isolieren der dermalen Hüllzellen aus einer Hautgewebeprobe, die von einem Tier entnommenen wurde, so dass die Zellen frei von Immunzellen sind, die an einer Abstoßungsreaktion eines Empfängertiers beteiligt sind.

11. Verfahren gemäß Anspruch 10, wobei die Immunzellen Leukozyten sind.

12. Verfahren gemäß Anspruch 10, wobei die Immunzellen Langerhans-Zellen sind.

13. Verfahren gemäß Anspruch 10, wobei die Immunzellen endotheliale Blutgefäßzellen sind.

14. Verfahren gemäß einem der Ansprüche 10-13, wobei die isolierten dermalen Hüllzellen durch Kryokonservierung gelagert werden.

15. Verfahren gemäß einem der Ansprüche 10-14, wobei die isolierten dermalen Hüllzellen von einem weiblichen Tier stammen.

16. Verfahren gemäß einem der Ansprüche 10-14, wobei die isolierten dermalen Hüllzellen von einem männlichen Tier stammen.

17. Verfahren gemäß einem der Ansprüche 10-16, wobei das Tier menschlich ist.

18. Verfahren gemäß Anspruch 10, wobei die Hautprobe eine Stanzbiopsie ist und die dermalen Hüllzellen durch ein Verfahren isoliert werden, das umfasst:
i) Schnelle Abtragung von Bulbusenden der Haarfollikel von der Stanzbiopsie;
ii) Ausstülpen und Aufdecken der epidermalen Anteile, die abgekratzt und verworfen werden;
iii) Trennung von dermaler Hülle/Zellen und dermalen Papillen;
iv) dermales Hüllgewebe/Zellen werden gereinigt; und optional;
v) dermales Hüllgewebe/Zellen werden getrennt in Zellkulturmedium gesammelt.

## Revendications

1. Utilisation de cellules de gaine dermique isolées pour la fabrication d'un médicament destiné à être utilisé dans le traitement d'états chez un animal receveur qui bénéficierait d'un remplacement et/ou d'une augmentation de cheveux.

2. Utilisation selon la revendication 1, dans laquelle les cellules de gaine dermique isolées et l'animal receveur sont du même genre.

3. Utilisation selon la revendication 1, dans laquelle les cellules de gaine dermique isolées et l'animal receveur sont de genres différents.

4. Utilisation selon la revendication 3, dans laquelle les cellules de gaine dermique isolées sont mâles et l'animal receveur est femelle.

5. Utilisation selon la revendication 3, dans laquelle les cellules de gaine dermique isolées sont femelles et l'animal receveur est mâle.

6. Utilisation selon l'une quelconque des revendications 1 à 5, dans laquelle l'animal receveur est humain.

7. Utilisation de cellules de gaine dermique isolées pour la fabrication d'un médicament pour le traitement de l'alopécie.

8. Utilisation de cellules de gaine dermique isolées pour la fabrication d'un médicament pour le traitement de la perte des cheveux par suite d'une cancérothérapie.

9. Utilisation de cellules de gaine dermique isolées pour le traitement cosmétique de la perte des cheveux.

10. Procédé pour isoler des cellules de gaine dermique présentant un privilège immunitaire comprenant l'isolement de cellules de gaine dermique à partir d'un échantillon de tissu cutané retiré d'un animal de telle sorte que les cellules sont dépourvues de cellules immunitaires mises en jeu dans une réponse de rejet de l'animal receveur.

11. Procédé selon la revendication 10, dans lequel les cellules immunitaires sont des leucocytes.

12. Procédé selon la revendication 10, dans lequel les cellules immunitaires sont des cellules de Langerhans.

13. Procédé selon la revendication 10, dans lequel les cellules immunitaires sont des cellules endothéliales de vaisseaux sanguins.

14. Procédé selon l'une quelconque des revendications 10 à 13, dans lequel les cellules de gaine dermique isolées sont stockées par cryoconservation.

15. Procédé selon l'une quelconque des revendications 10 à 14, dans lequel les cellules de gaine dermique isolées proviennent d'un animal femelle.

16. Procédé selon l'une quelconque des revendications 10 à 14, dans lequel les cellules de gaine dermique isolées proviennent d'un animal mâle.

17. Procédé selon l'une quelconque des revendications 10 à 16, dans lequel l'animal est humain.

18. Procédé selon la revendication 10, dans lequel ledit échantillon de peau est une biopsie à l'emporte-pièce et lesdites cellules de gaine dermique sont isolées par un procédé comprenant :
i) une amputation rapide de bulbes terminaux de follicules capillaires à partir de ladite biopsie à l'emporte-pièce ;
ii) une éversion et une exposition de composants épidermiques qui sont retirés par grattage et mis au rebut ;
iii) une séparation de tissu/cellules de gaine dermique et de papilles dermiques ;
iv) le tissu/les cellules de gaine dermique sont nettoyés ; et facultativement,
v) le tissu/les cellules de gaine dermique sont groupés de manière séparée dans un milieu de culture.
